# EUROPEAN PATENT APPLICATION

(11) **EP 3 992 146 A1**
(43) Date of publication of application: **04.05.2022**
(21) Application number: 20831279.3
(22) Date of filing: 22.06.2020
(51) Int. Cl.: C01B 32/77

(54) **METHOD FOR PRODUCING CARBONYL SULFIDE**

(30) Priority: 27.06.2019 JP 2019119959
(71) Applicant: Zeon Corporation, Tokyo 100-8246 (JP)
(72) Inventor: MATSUURA, Go, Tokyo 100-8246 (JP)
(74) Representative: Gerauer, Marc Philippé
(86) International application number: PCT/JP2020/024447
(87) International publication number: WO 2020/262319

(57) **Abstract**

Provided is a production method that enables production of carbonyl sulfide by a gas phase flow method without using a catalyst. The method of producing carbonyl sulfide includes causing electrical discharge of a feedstock gas containing starting substances that include CS₂ and one or more selected from the group consisting of CO₂, CO, O₂, and O₃ while in a continuous flow state and then causing release to outside of an electrical discharge zone.

## Description

### TECHNICAL FIELD

The present disclosure relates to a method of producing carbonyl sulfide.

### BACKGROUND

Carbonyl sulfide (COS) is known to be useful as a gas for etching a carbon hard mask or the like in a semiconductor production process.

Known methods for producing carbonyl sulfide in a gas phase include a method in which carbon dioxide gas and carbon disulfide are reacted in the presence of a catalyst (Patent Literature (PTL) 1 and 2) and a method in which sulfur and carbon monoxide are reacted in the presence of a catalyst.

### CITATION LIST

### Patent Literature

PTL 1: JP-S47-40632B
PTL 2: US3409399A
PTL 3: JP-S52-131993A

### SUMMARY

### (Technical Problem)

However, the methods of producing carbonyl sulfide described above each use a catalyst, and continuous production thereby has been difficult due to reduction of yield accompanying reduction of catalyst activity, for example.

Accordingly, an object of the present disclosure is to provide a production method that enables production of carbonyl sulfide by a gas phase flow method without using a catalyst.

### (Solution to Problem)

The inventor conducted diligent studies to achieve the object set forth above and discovered that carbonyl sulfide can be obtained by causing electrical discharge of a specific feedstock gas while in a continuous flow state and then continuously causing release to outside of the electrical discharge zone. In this manner, the inventor completed the present disclosure.

The present disclosure aims to advantageously solve the problem set forth above and relates to a method of producing carbonyl sulfide that comprises causing electrical discharge of a feedstock gas containing starting substances that include CS₂ and one or more selected from the group consisting of CO₂, CO, O₂, and O₃ while in a continuous flow state and then continuously causing release to outside of an electrical discharge zone.

In the presently disclosed method of producing carbonyl sulfide, electrical discharge of the feedstock gas while in a continuous flow state causes the starting substances contained in the feedstock gas, which include CS₂ and one or more selected from the group consisting of CO₂, CO, O₂, and O₃, to be converted to active species that can serve as precursors for COS, whereas the subsequent continuous release to outside of the electrical discharge zone causes recombination of the active species to thereby produce carbonyl sulfide. In this manner, it is possible to produce carbonyl sulfide (COS) by a gas phase flow method according to the present disclosure without using a catalyst.

The starting substances include CS₂ and one or more selected from the group consisting of CO₂, CO, O₂, and O₃. The combined use of these substances enables suitable generation of CS active species, CO active species, and active species formed of elemental oxygen that can serve as precursors for COS.

The presently disclosed method is advantageous because COS can be produced without using a catalyst.

In the presently disclosed method, 0.3 kW or more of electrical power can be supplied to the feedstock gas to cause electrical discharge, and electrical discharge of the feedstock gas can be caused to occur between electrodes having an interelectrode distance of 1 cm or more. The above are advantageous in terms of enabling stable conversion of active species that can serve as precursors for COS.

In the presently disclosed method, a ratio of volume of the CS₂ relative to total volume of the one or more selected from the group consisting of CO₂, CO, O₂, and O₃ is preferably 0.02 or more. A ratio that is within this range makes it possible to sufficiently obtain COS.

In the presently disclosed method, a ratio of volume of the CS₂ relative to volume of the feedstock gas is preferably not less than 0.02 and not more than 0.3. A ratio that is within this range makes it possible to sufficiently obtain COS.

### (Advantageous Effect)

According to the present disclosure, it is possible to produce carbonyl sulfide by a gas phase flow method without using a catalyst. The presently disclosed production method makes it possible to avoid reduction of yield caused by reduction of catalyst activity and enables continuous production of carbonyl sulfide.

### DETAILED DESCRIPTION

The following provides a detailed description of embodiments of the present disclosure.

### [Feedstock gas]

The feedstock gas contains starting substances that include CS₂ and one or more selected from the group consisting of CO₂, CO, O₂, and O₃. A combination of CS₂ and CO₂ is more preferable in terms of efficiently obtaining COS, whereas a combination of CS₂ and O₂ is more preferable in terms of balance of feedstock conversion rate and selectivity.

A ratio of volume of the CS₂ relative to total volume of the one or more selected from the group consisting of CO₂, CO, O₂, and O₃ is preferably 0.02 or more in terms of selectivity of carbonyl sulfide. This ratio is more preferably 0.3 or more in terms of selectivity of COS. Moreover, this volume ratio is preferably 35 or less. A ratio that is within this range makes it possible to obtain good selectivity while also maintaining the feedstock conversion rate.

The feedstock gas may contain an inert gas. The inclusion of an inert gas makes it easy to achieve stable electrical discharge. The inert gas may be N₂, He, Ne, Ar, Xe, Kr, or the like, is preferably N₂, Ar, or He, and is more preferably N₂ or Ar. In a case in which an inert gas is used, just one type of inert gas may be used, or two or more types of inert gases may be used in combination.

The proportional content of an inert gas in the feedstock gas in a case in which an inert gas is used can be 99.9 volume% or less, and is preferably 99 volume% or less. The proportional content of an inert gas may be 0 volume%.

Besides the starting substances and the optional inert gas, the feedstock gas can contain impurities that are unavoidably mixed into the feedstock gas from the surrounding environment. Examples of such impurities include moisture. The feedstock gas can be a gas that is composed of the starting substances and unavoidable impurities.

A ratio of volume of the CS₂ relative to the feedstock gas is preferably 0.02 or more. Moreover, this volume ratio is preferably 0.3 or less. A ratio that is within this range makes it possible to sufficiently obtain COS.

The feedstock gas should contain the starting substances and the optional inert gas when electrical discharge thereof is caused to occur. For example, in order to cause electrical discharge, a gas phase flow reactor having an electrical discharge mechanism (hereinafter, also referred to simply as a "gas phase flow reactor") may be supplied with the starting substances and the optional inert gas as separately supplied gases so as to provide the feedstock gas, may be supplied with a gas obtained through premixing of all of the starting substances and the optional inert gas so as to provide the feedstock gas, or may be separately supplied with a gas obtained by premixing a portion of the starting substances and the optional inert gas and a gas of the remainder thereof so as to provide the feedstock gas.

Each of the starting substances is a gas or liquid in a standard state. Although each of the starting substances can be supplied to the gas phase flow reactor as a gas without providing a separate vaporization chamber or the like, in the case of a liquid, it is preferable that the starting substance is supplied to the gas phase flow reactor after being vaporized in a separately provided vaporization chamber. This supply can be performed continuously. Control of the supply flow rate can be performed using a mass flow controller or the like.

For example, a starting substance in a liquid state can be vaporized by introducing the liquid-state starting substance into a vaporization chamber held at a temperature and pressure at which sufficient vaporization of the starting substance occurs. The temperature and pressure of the vaporization chamber are preferably held at a temperature and pressure that enable instantaneous vaporization of the starting substance. The use of such a vaporization chamber makes it possible to continuously introduce a starting substance into the vaporization chamber as a liquid, cause instantaneous vaporization thereof in the vaporization chamber, and then continuously supply the starting substance into the gas phase flow reactor as a gas. In the case of a starting substance having a solid state, the starting substance may be converted to a liquid by heating and subsequently be introduced into the vaporization chamber, or may be directly sublimated in the vaporization chamber and then continuously supplied into the gas phase flow reactor as a gas.

Control of the supply flow rate can be performed by using a mass flow controller or the like to control gas that has been vaporized in the vaporization chamber or can be performed by using a liquid mass flow controller or the like to control continuous introduction of a starting substance into the vaporization chamber in a liquid state. A starting substance that has been vaporized may be diluted with an inert gas or the like when it is introduced into the gas phase flow reactor.

The space velocity when the feedstock gas is caused to flow in the gas phase flow reactor is not specifically limited but is preferably 0.01 hr⁻¹ or more, more preferably 0.1 hr⁻¹ or more, and even more preferably 0.3 hr⁻¹ or more, and is preferably 100,000 hr⁻¹ or less, more preferably 50,000 hr⁻¹ or less, and even more preferably 10,000 hr⁻¹ or less. A space velocity that is within any of the ranges set forth above makes it possible to avoid complication of electrical discharge and enables efficient production of the target substance without reduction of productivity.

### [Electrical discharge]

The feedstock gas is caused to undergo electrical discharge in the gas phase flow reactor to thereby generate active species that can serve as precursors for COS from the feedstock gas. The electrical discharge can be caused by supplying electrical power to the electrical discharge mechanism of the gas phase flow reactor. For example, the supply of electrical power can cause electrical discharge between electrodes installed inside the gas phase flow reactor.

The electrical power supplied when causing electrical discharge is preferably 0.3 kW or more. When the supplied electrical power is within this range, electrical discharge can be stabilized, and the target substance can be efficiently produced. Moreover, the supplied electrical power is preferably 100 kW or less. When the supplied electrical power is within this range, it is possible to avoid reaction tube blocking caused by soot formation of the feedstock gas, for example, and to stably produce the target substance. The supplied electrical power is more preferably 0.5 kW or more, and is more preferably 60 kW or less.

The electrical discharge can be generated between electrodes, with an interelectrode distance of 1 cm or more being preferable in the presently disclosed method. Moreover, the interelectrode distance is preferably 100 cm or less. An interelectrode distance that is within this range enables stable electrical discharge.

The method of electrical discharge of the feedstock gas can be a method that includes electrodes for applying a voltage that causes electrical discharge to occur. Examples of methods that can be used include high-frequency discharge, microwave discharge, dielectric barrier discharge, glow discharge, arc discharge, and corona discharge. High-frequency discharge, glow discharge, and arc discharge are preferable in terms of stability of electrical discharge and quantity of processed gas.

Although no specific limitations are placed on the pressure (absolute pressure) during electrical discharge so long as it is a pressure at which electrical discharge of the feedstock gas can occur in the adopted electrical discharge method, the pressure is preferably 1 PaA or higher, and more preferably 5 PaA or higher, and is preferably 1 MPaA or lower, and more preferably 0.5 MpaA or lower. A pressure (absolute pressure) that is within any of the ranges set forth above enables efficient production of the target substance.

### [Target substance]

The feedstock gas that has undergone electrical discharge is continuously released from the electrical discharge zone to thereby cause recombination of generated active species and produce carbonyl sulfide that is a target substance. The continuous release can be performed with a space velocity corresponding to the continuous flow of the feedstock gas.

The electrical discharge zone is a space in which electrical discharge of the feedstock gas is caused to occur. For example, in the case of a gas phase flow reactor including parallel plate electrodes as an electrical discharge mechanism, the electrical discharge zone is a space where electrical discharge is generated between the electrodes. Moreover, release to outside of the electrical discharge zone means exiting from the aforementioned space to outside of the space.

Note that after gas that has undergone electrical discharge has been released from the electrical discharge zone and has exited the gas phase flow reactor, the gas may be introduced into a heat exchanger and may be cooled. The mechanism of the heat exchanger is not specifically limited and may be air cooling, water cooling, or the like. Since the cooled product may contain substances other than carbonyl sulfide, the product may be subjected to an optionally performed separation and purification step so as to separate and purify the carbonyl sulfide. Examples of separation and purification methods that can be used include distillation, absorption by a solution or the like, and membrane separation.

### EXAMPLES

The present disclosure is described in more detail below through examples. However, the present disclosure is not limited by these examples.

### (Example 1)

CS₂ and CO₂ as starting substances and Ar as an inert gas were supplied at flow rates of 70 sccm, 2 sccm, and 228 sccm, respectively, to a gas phase flow reaction tube made of metal in which parallel-plate electrode capable of high-frequency discharge was installed (frequency: 60 MHz, capacity: 35 L; interelectrode distance: 3.5 cm).

Electrical discharge was caused to occur through 500 W of supplied electrical power while holding the mixed gas at 10 PaA (absolute pressure) inside the reaction tube. Gas was continuously released from the reaction tube and was trapped in an aluminum bag.

The trapped gas was analyzed by gas chromatography-mass spectrometry (GC-MS) (Agilent 7890A produced by Agilent Technologies, Inc.). The molar conversion rate of CO₂ was determined from area values for components in GC-MS that were obtained through the analysis, and this molar conversion rate was taken to be the feedstock conversion rate. In addition, the molar selectivity of each component in the product was determined from the aforementioned area values. The results are shown in Table 1.

### (Example 2)

Example 2 is the same as Example 1 with the exception that the flow rates of CS₂, CO₂, and Ar were changed to 50 sccm, 10 sccm, and 240 sccm, respectively. The results are shown in Table 1. The feedstock conversion rate in this example is the molar conversion rate of CO₂ determined from area values for components in GC-MS.

### (Example 3)

Example 3 is the same as Example 2 with the exception that the flow rates of CS₂ and Ar were changed to 30 sccm and 260 sccm, respectively. The results are shown in Table 1. The feedstock conversion rate in this example is the molar conversion rate of CO₂ determined from area values for components in GC-MS.

### (Example 4)

Example 4 is the same as Example 2 with the exception that the flow rates of CS₂ and Ar were changed to 10 sccm and 280 sccm, respectively. The results are shown in Table 1. The feedstock conversion rate in this example is the molar conversion rate of CS₂ determined from area values for components in GC-MS.

### (Example 5)

Example 5 is the same as Example 4 with the exception that the flow rates of CO₂ and Ar were changed to 30 sccm and 260 sccm, respectively. The results are shown in Table 1. The feedstock conversion rate in this example is the molar conversion rate of CS₂ determined from area values for components in GC-MS.

### (Example 6)

Example 6 is the same as Example 4 with the exception that the flow rates of CO₂ and Ar were changed to 100 sccm and 190 sccm, respectively. The results are shown in Table 1. The feedstock conversion rate in this example is the molar conversion rate of CS₂ determined from area values for components in GC-MS.

### (Example 7)

Example 7 is the same as Example 3 with the exception that Ar was changed to N₂. The results are shown in Table 1. The feedstock conversion rate in this example is the molar conversion rate of CO₂ determined from area values for components in GC-MS.

### (Example 8)

Example 8 is the same as Example 5 with the exception that Ar was changed to N₂. The results are shown in Table 1. The feedstock conversion rate in this example is the molar conversion rate of CS₂ determined from area values for components in GC-MS.

### (Example 9)

Example 9 is the same as Example 1 with the exception that the flow rates of CS₂ and CO₂ were set as 70 sccm and 230 sccm, respectively, and an inert gas was not used. The results are shown in Table 1. The feedstock conversion rate in this example is the molar conversion rate of CS₂ determined from area values for components in GC-MS.

### (Example 10)

Example 10 is the same as Example 9 with the exception that the supplied electrical power was changed to 2,000 W. The results are shown in Table 1. The feedstock conversion rate in this example is the molar conversion rate of CS₂ determined from area values for components in GC-MS.

### (Example 11)

Example 11 is the same as Example 9 with the exception that the flow rates of CS₂ and CO₂ were changed to 5 sccm and 295 sccm, respectively. The results are shown in Table 1. The feedstock conversion rate in this example is the molar conversion rate of CS₂ determined from area values for components in GC-MS.

### (Example 12)

Example 12 is the same as Example 2 with the exception that CO₂ was changed to O₂. The results are shown in Table 2. The feedstock conversion rate in this example is the molar conversion rate of O₂ determined from area values for components in GC-MS.

### (Example 13)

Example 13 is the same as Example 3 with the exception that CO₂ was changed to O₂. The results are shown in Table 2. The feedstock conversion rate in this example is the molar conversion rate of O₂ determined from area values for components in GC-MS.

### (Example 14)

Example 14 is the same as Example 4 with the exception that CO₂ was changed to O₂. The results are shown in Table 2. The feedstock conversion rate in this example is the molar conversion rate of CS₂ determined from area values for components in GC-MS.

### (Example 15)

Example 15 is the same as Example 2 with the exception that CO₂ was changed to CO. The results are shown in Table 3. The feedstock conversion rate in this example is the molar conversion rate of CO determined from area values for components in GC-MS.

### (Example 16)

Example 16 is the same as Example 4 with the exception that CO₂ was changed to CO. The results are shown in Table 3. The feedstock conversion rate in this example is the molar conversion rate of CS₂ determined from area values for components in GC-MS.

### (Example 17)

Example 17 is the same as Example 5 with the exception that CO₂ was changed to CO. The results are shown in Table 3. The feedstock conversion rate in this example is the molar conversion rate of CS₂ determined from area values for components in GC-MS.

**Table 1**

| | | | | | | | | | | | | Product selectivity [mol%] | | | |
|---|---|---|---|---|---|---|---|---|---|---|---|---|---|---|---|
| Example | Starting substance (1) | Flow rate [sccm] | Starting substance (2) | Flow rate [sccm] | Inert gas | Flow rate [sccm] | Volume ratio (starting substance (1)/ starting substance (2)) | Volume ratio (starting substance (1)/ overall gas) | Electrical power (kW) | Space velocity [hr⁻¹] | Feedstock conversion rate [mol%] | H₂S^{∗} | COS | SO₂ | Other |
| Example 1 | CS₂ | 70 | CO₂ | 2 | Ar | 228 | 35.0 | 0.23 | 0.50 | 0.5 | 1.9% | 0.0% | 100.0% | 0.0% | 0.0% |
| Example 2 | | 50 | | 10 | | 240 | 5.00 | 0.17 | 0.50 | 0.5 | 1.9% | 0.0% | 100.0% | 0.0% | 0.0% |
| Example 3 | | 30 | | 10 | | 260 | 3.00 | 0.10 | 0.50 | 0.5 | 1.8% | 0.0% | 100.0% | 0.0% | 0.0% |
| Example 4 | | 10 | | 10 | | 280 | 1.00 | 0.03 | 0.50 | 0.5 | 5.3% | 7.6% | 29.5% | 62.9% | 0.0% |
| Example 5 | | 10 | | 30 | | 260 | 0.33 | 0.03 | 0.50 | 0.5 | 38.7% | 3.5% | 8.0% | 88.6% | 0.0% |
| Example 6 | | 10 | | 100 | | 190 | 0.10 | 0.03 | 0.50 | 0.5 | 70.1% | 0.4% | 2.5% | 97.1% | 0.0% |
| Example 7 | | 30 | | 10 | N₂ | 260 | 3.00 | 0.10 | 0.50 | 0.5 | 3.2% | 0.0% | 22.8% | 0.0% | 77.2% |
| Example 8 | | 10 | | 30 | | 260 | 0.33 | 0.03 | 0.50 | 0.5 | 15.7% | 1.4% | 4.6% | 93.3% | 0.6% |
| Example 9 | | 70 | | 230 | - | - | 0.30 | 0.23 | 0.50 | 0.5 | 8.9% | 0.0% | 49.4% | 50.6% | 0.0% |
| Example 10 | | 70 | | 230 | - | - | 0.30 | 0.23 | 2.00 | 0.5 | 20.5% | 0.0% | 53.1% | 46.9% | 0.0% |
| Example 11 | | 5 | | 295 | - | - | 0.02 | 0.02 | 0.50 | 0.5 | 11.6% | 0.0% | 22.2% | 77.8% | 0.0% |

| | | | | | | | | | | | | | | | |
|---|---|---|---|---|---|---|---|---|---|---|---|---|---|---|---|
| ^{∗} Assumed to be due to moisture unavoidably contained in feedstock gas | | | | | | | | | | | | | | | |

**Table 2**

| | | | | | | | | | | | | Product selectivity [mol%] | | | | |
|---|---|---|---|---|---|---|---|---|---|---|---|---|---|---|---|---|
| Example | Starting substance (1) | Flow rate [sccm] | Starting substance (2) | Flow rate [sccm] | Inert gas | Flow rate [sccm] | Volume ratio (starting substance (1)/ starting substance (2)) | Volume ratio (starting substance (1)/ overall gas) | Electrical power (kW) | Space velocity [hr⁻¹] | Feedstock conversion rate [mol%] | CO₂ | H₂S^{∗} | COS | SO₂ | Other |
| Example 12 | CS₂ | 50 | O₂ | 10 | Ar | 240 | 5.00 | 0.20 | 0.50 | 0.5 | 5.7% | 44.9% | 0.0% | 55.1% | 0.0% | 0.0% |
| Example 13 | | 30 | | 10 | | 260 | 3.00 | 0.13 | 0.50 | 0.5 | 9.2% | 54.6% | 0.0% | 45.4% | 0.0% | 0.0% |
| Example 14 | | 10 | | 10 | | 280 | 1.00 | 0.07 | 0.50 | 0.5 | 43.9% | 29.7% | 1.9% | 4.2% | 64.2% | 0.0% |

| | | | | | | | | | | | | | | | | |
|---|---|---|---|---|---|---|---|---|---|---|---|---|---|---|---|---|
| ^{∗} Assumed to be due to moisture unavoidably contained in feedstock gas | | | | | | | | | | | | | | | | |

**Table 3**

| | | | | | | | | | | | | Product selectivity [mol%] | | | | |
|---|---|---|---|---|---|---|---|---|---|---|---|---|---|---|---|---|
| Example | Starting substance (1) | Flow rate [sccm] | Starting substance (2) | Flow rate [sccm] | Inert gas | Flow rate [sccm] | Volume ratio (starting substance (1)/ starting substance (2)) | Volume ratio (starting substance (1)/ overall gas) | Electrical power (kW) | Space velocity [hr⁻¹] | Feedstock conversion rate [mol%] | CO₂ | H₂S^{∗} | COS | SO₂ | Other |
| Example 15 | CS₂ | 50 | CO | 10 | Ar | 240 | 5.00 | 0.20 | 0.50 | 0.5 | 6.2% | 81.0% | 0.0% | 19.0% | 0.0% | 0.0% |
| Example 16 | | 10 | | 10 | | 280 | 1.00 | 0.07 | 0.50 | 0.5 | 8.8% | 88.8% | 0.0% | 11.2% | 0.0% | 0.0% |
| Example 17 | | 10 | | 30 | | 260 | 0.33 | 0.13 | 0.50 | 0.5 | 12.5% | 88.6% | 0.0% | 11.4% | 0.0% | 0.0% |

| | | | | | | | | | | | | | | | | |
|---|---|---|---|---|---|---|---|---|---|---|---|---|---|---|---|---|
| ^{∗} Assumed to be due to moisture unavoidably contained in feedstock gas | | | | | | | | | | | | | | | | |

It can be seen from Tables 1 to 3 that carbonyl sulfide could be produced without using a catalyst in the examples. It can also be seen that selectivity of COS increases with increasing volume proportion of CS₂ among the starting substances. Examples 9 to 11 demonstrate that good carbonyl sulfide selectivity can be achieved even when an inert gas is not used.

### INDUSTRIAL APPLICABILITY

According to the present disclosure, it is possible to produce carbonyl sulfide by a gas phase flow method without using a catalyst. The presently disclosed production method makes it possible to avoid reduction of yield caused by reduction of catalyst activity, enables continuous production of carbonyl sulfide, and has high industrial applicability.

## Claims

1. A method of producing carbonyl sulfide comprising causing electrical discharge of a feedstock gas containing starting substances that include CS₂ and one or more selected from the group consisting of CO₂, CO, O₂, and O₃ while in a continuous flow state and then causing release to outside of an electrical discharge zone.

2. The method of producing carbonyl sulfide according to claim 1, wherein a catalyst is not used.

3. The method of producing carbonyl sulfide according to claim 1 or 2, wherein 0.3 kW or more of electrical power is supplied when causing electrical discharge of the feedstock gas.

4. The method of producing carbonyl sulfide according to any one of claims 1 to 3, wherein electrical discharge of the feedstock gas is caused between electrodes having an interelectrode distance of 1 cm or more.

5. The method of producing carbonyl sulfide according to any one of claims 1 to 4, wherein a ratio of volume of the CS₂ relative to total volume of the one or more selected from the group consisting of CO₂, CO, O₂, and O₃ is 0.02 or more.

6. The method of producing carbonyl sulfide according to any one of claims 1 to 5, wherein a ratio of volume of the CS₂ relative to volume of the feedstock gas is not less than 0.02 and not more than 0.3.
